# EUROPEAN PATENT SPECIFICATION

(11) **EP 1 996 132 B1**
(45) Date of publication and mention of the grant of the patent: **26.10.2011**
(21) Application number: 07752917.0
(22) Date of filing: 12.03.2007
(51) Int. Cl.: A61F 2/88

(54) **ENDOPROSTHESIS HAVING MULTIPLE HELICALLY WOUND FLEXIBLE FRAMEWORK ELEMENTS**
ENDOPROTHESE MIT MEHREREN SPIRALFÖRMIG GEWUNDENEN FLEXIBLEN STRUKTURELEMENTEN
ENDOPROTHÈSE À ÉLÉMENTS MULTIPLE HÉLICOÏDAUX DE STRUCTURE FLEXIBLE POUR LÉSION

(30) Priority: 17.03.2006 US 378048
(43) Date of publication of application: 03.12.2008
(73) Proprietor: Gore Enterprise Holdings, Inc., Newark, DE 19714-9206 (US)
(72) Inventor: CULLY, Edward, H., Flagstaff, AZ 86004 (US); HUPPENTHAL, Joseph, A., Flagstaff, AZ 86004 (US); VONESH, Michael, J., Flagstaff, AZ 86004 (US)
(74) Representative: Shanks, Andrew
(86) International application number: PCT/US2007/006252
(87) International publication number: WO 2007/109007

(56) References cited:
- EP-A- 1 036 551
- WO-A-00/42949
- WO-A-02/39906
- WO-A-93/13825
- US-A- 5 876 432
- US-A1- 2005 080 481

## Description

### FIELD OF THE INVENTION

The present invention relates to the field of medical devices. More particularly, the invention is directed to implantable stent devices, including stent-grafts, having multiple flexible framework elements.

### BACKGROUND OF THE INVENTION

Implantable stents and stent-grafts (*i.e.,* stents provided with graft coverings) have been used for some years in a variety of different body conduits as means for maintaining the patency of the body conduit within which they were implanted. While the primary application has been in the arterial vascular system, these devices also have been used in the venous system and in other body conduits such as the esophagus. For ease of deployment, these stent and stent-graft devices are typically provided in a conformation having a diameter smaller than that of the body conduit into which the devices are inserted. Once delivered to a desired site with a suitable delivery system, the devices are deployed and implanted by an appropriate method that results in an increase in the diameter of the stent device. The device diameter is increased by inflating a catheter balloon located inside the device or by removing a mechanical restraint from the device and allowing the device to self-expand. In most cases, the devices are secured to the luminal wall of the body conduit with an interference fit. Some devices have anchoring means that engage the luminal wall to secure the device in place.

Various stents have been described that are made from helically wound filaments. The filaments are usually made of a metallic alloy, such as nitinol metal or stainless steel. A self-expanding endovascular stent formed from a stainless steel wire is described in U.S. Patent No. 5,507,767, issued to Maeda et al. The stainless steel wire is formed into a zigzag pattern with an eyelet at each reverse bend in the pattern. The length of wire between adjacent zigzag-bends in the wire can be varied to provide a device customized to better conform to a patient's particular vascular anatomy. The single zigzag-shaped wire is helically wound around a central axis to define a tubular shape. The zlgzag-shaped wire is maintained in the tubular shape with a single filament connecting adjacent eyelets of the zigzag-shaped wire. While this design is said by Maeda et al. to provide an elongated self-expanding stent having substantially uniform expansile force along the length of the stent, deficiencies remain with the design. For example, the stent undergoes significant changes in axial length as it self-expands from a compacted configuration to its deployed configuration. This fore-shortening may introduce an unacceptable degree of uncertainty into the process of precisely placing the device in a patient's vasculature. The fore-shortening would also make placement of a flexible sleeve on the device problematical.

Lau et al., U.S. Patent No. 5,876,432, also describe a self-expanding metallic stent made of a single helically wound undulating member. The helically wound undulating member is maintained in a tubular shape with a single coupling member that extends through undulations of adjacent turns of the helically wound member. Unlike the Maeda et al. stent, the single coupling member of the Lau et al. device is movable along the undulations and not confined to eyelets located at turns in the undulating member. A covering can be applied to the Lau et al. stent framework to form a stent-graft.

Neither of these devices uses two or more separate structural elements helically disposed around a common central axis to form the device. Nor do they use two or more linkage to connect the separate structural elements together. A stent device having two or more separate structural or linkage elements would provide a variety of design and material options to a practitioner. Each of these elements could be made of a different composition. The elements could also have different cross-sectional shapes and/or different material properties.

There is a need, therefore, for a stent with two or more separate structural elements helically disposed around a common central axis and connected with two or more separate linkage elements. There is also a need for a stent-graft utilizing such a structural framework.

### SUMMARY OF THE INVENTION

The present invention is directed to implantable medical devices for use within a body conduit or luminal space. The invention serves primarily as a mechanical reinforcement for the conduit or space. The mechanical support can be provided permanently or on a temporary basis. Optionally, the invention can include releasable compositions.

The present invention can be made of materials having different compositions and geometries. A component made of one material or geometry can influence and cooperate with the properties of another component in the invention. For example, an expandable stent or expandable stent-graft can be made with both shape-memory metallic framework elements and plastically-deformable metallic framework elements. By varying the ratio and/or conformation of one type of framework element with respect to another type of framework element, the composite mechanical properties of the expandable device can be adjusted. In embodiments having more shape-memory metallic framework elements than plastically-deformable metallic framework elements, for example, the invention would have self-expanding properties imparted by the shape-memory metallic framework elements and a high degree of radial strength imparted by the plastically-deformable metallic framework elements. In embodiments having fewer shape-memory metallic framework elements than plastically-deformable metallic framework elements, the expandable device could be formed into a collapsed configuration and so remain without the need for a separate constraining sheath. In use, the compacted invention would be radially expanded at an Implantation site with an inflatable balloon, or other means of deployment.

In a preferred embodiment, the invention has a generally tubular shape and is made of two or more structural, or framework, elements, each helically disposed around a common central axis running the length of the invention. Preferably, the two helically formed framework elements are serpentine windings with apices in the form of small radius bends in wire elements, with alternating apices pointing in opposing directions, running along at least a portion of the framework element. In addition to being simple bends, or turns, in the framework element, the apices can be in a particular shape or form. The framework elements are arranged together in the helical windings such that apices of a first framework element are positioned adjacent apices of a second framework element. Adjacent apices of the first and second framework elements are connected together with a first or second flexible linkage element The first and second flexible linkage elements each link a distinct set of adjacent apices. Each set of adjacent apices establishes a distinct pathway or course along which the flexible linkage element is placed. The courses are determined by the relative positions of the apices of the first and second framework elements and the pattern the adjacent apices form along the length of the invention. The flexible linkage elements do not cause the invention to assume a tubular configuration. Rather, the flexible linkage elements assist in maintaining the flexible framework elements in proper relationship and orientation.

Accordingly, one embodiment of the present invention is an implantable medical device comprising a first framework element having a length and a series of apices oriented in opposite directions along the length of the first framework element, wherein said first framework element is helically disposed around a common central axis, a second framework element having a length and a series of apices oriented in opposite directions along the length of the second framework element, wherein said second framework element is helically disposed around the common central axis and positioned together with the first framework element so that apices of the first framework element and apices of the second framework element are located adjacent to one another, a first flexible linkage element linking the first framework element to the second framework element along a first course, and a second flexible linkage element linking the first framework element to the second framework element along a second course distinct from the first course.

Framework elements can be metallic or polymeric in composition. In some embodiments, the framework elements are made of different materials or materials of different dimensions. In other embodiments, the first framework element is made of a biocompatible metal having shape-memory properties, while the second framework element is made of a biocompatible metal having plastically-deformable properties. In other embodiments, the first framework element is made of a biocompatible metal and the second framework element made of a bioabsorbable material. The framework elements can have a polymeric coating or covering applied thereto and/or a biologically active composition or entity incorporated therewith. Accordingly, the composition, dimension, cross-sectional profile, and physical properties of individual framework elements can vary from element to element.

As with the framework elements, the flexible linkage elements can be made of biocompatible metallic alloys having shape-memory or plastically deformable properties. In addition to metallic alloys, the flexible linkage elements can be made of one or more polymeric materials. The polymeric materials can be non-bioabsorbable or bioabsorbable.

The materials of a flexible linkage element can be combined or used singly in a variety of forms such as monofilaments, braids, twisted filaments, ropes, or other configuration. Each of these forms can be wrapped, or covered, with an additional material. The flexible linkage elements can have a biologically active composition or entity incorporated therewith and/or have coatings applied thereto. The coatings can have one or more biologically active compositions or entities releasably incorporated therein.

When used as a medical device, the present invention can serve as a stent. A covering can be placed on at least a portion of the stent to form a stent-graft. In preferred stent-grafts, the covering spans space between adjacent framework elements. The covering may be external and/or internal to the framework elements. When internal and external coverings are provided, the coverings may be bonded together between the framework elements. Accordingly, another embodiment of the present invention is an implantable medical device comprising a first framework element having a length and a series of apices oriented in opposite directions along the length of the first framework element, wherein said first framework element is helically disposed around a common central axis, a second framework element having a length and a series of apices oriented in opposite directions along the length of the second framework element, wherein said second framework element is helically disposed around the common central axis and positioned together with the first framework element so that apices of the first framework element and apices of the second framework element are located adjacent to one another, a first flexible linkage element linking the first framework element to the second framework element along a first course, a second flexible linkage element linking the first framework element to the second framework element along a second course distinct from the first course, and a covering placed on at least a portion of said device.

The above is a brief description of some deficiencies in the prior art and the advantages and aspects of the present invention. Other features, advantages, and embodiments of the invention will be apparent to those skilled in the art from the following description, accompanying drawings, and appended claims.

### BRIEF DESCRIPTION OF THE DRAWINGS

Figure 1 is an illustration of a side view of a first framework element of the present invention helically disposed around a common central axis of a jig.
Figure 2 is an illustration of a side view of a second framework element of the present invention helically disposed around a common central axis of a jig.
Figure 3 is an illustration of a side view of a first framework element and a second framework element both helically disposed around a common central axis on a jig.
Figure 4 is an illustration of a side view of a first framework element and a second framework element connected together with a first linkage element along a first course.
Figure 5 is an illustration of a side view of a first framework element and a second framework element connected together with a second linkage element along a second course.
Figure 6 is an illustration of a side view of the present invention on a mandrel.
Figure 7 is an illustration of a side view of the present invention on a mandrel.
Figure 8 is an illustration of a stent-graft of the present invention.
Figure 9 is an illustration of a framework element in cross-section.
Figure 10 is an illustration of a framework element in cross-section.
Figure 11 is an illustration of a framework element in cross-section.
Figure 12 is an illustration of a framework element in cross-section.
Figure 13 is an illustration of a framework element in cross-section.
Figure 14 is an illustration of a linkage element in cross-section.
Figure 15 is an illustration of a linkage element in cross-section.
Figure 16 is an illustration of a linkage element in cross-section.
Figure 17 is an illustration of a linkage element in cross-section.
Figure 18 is an illustration of flexible framework elements of the present invention.
Figure 19 is an illustration of flexible framework elements and a first course of flexible linkage elements of the present invention.
Figure 20 is an illustration of flexible framework elements and a second course of flexible linkage elements of the present invention.
Figure 21 is an illustration of first flexible framework elements, second flexible framework elements, a first course of flexible linkage elements, and a second course of flexible linkage elements of the present invention.
Figure 22 is an illustration of a perspective view of flexible framework elements of the present invention on a mandrel.
Figure 23 is an illustration of a perspective view of flexible framework elements of the present invention partially removed from a mandrel and flexible linkage elements incorporated therewith.
Figure 24 is an illustration of a perspective view of present invention in the form of a stent-graft.
Figure 25 is an illustration of a perspective view of flexible framework elements of the present invention on a mandrel.
Figure 26 is an illustration of a perspective view of flexible framework elements of the present invention partially removed from a mandrel and flexible linkage elements incorporated therewith.
Figure 27 is an illustration of a perspective view of present invention in the form of a stent-graft.

### DETAILED DESCRIPTION OF THE INVENTION

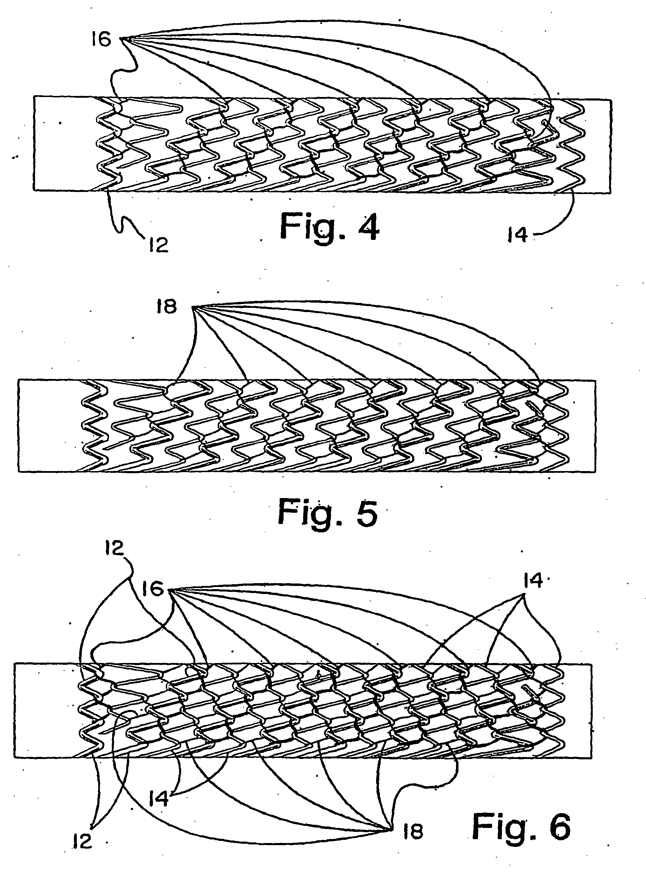

The present invention is directed to devices made of at least two flexible framework elements (12, 14) that can each vary in composition, form, physical properties, and dimension (Figure 6). The two or more flexible framework elements are connected together with two or more separate flexible linkage elements (16,18). Each separate flexible linkage element follows a distinct path as it courses through and connects the flexible framework elements of the invention. In a preferred embodiment, the combined flexible framework elements are in a tubular form and can function as a stent. In another preferred embodiment, the tubular form is covered and/or lined with a biocompatible material and can function as a stent-graft. Each component of the present invention can be made of biocompatible metallic materials, including alloys, and/or biocompatible polymeric materials, including non-bioabsorbable materials and bioabsorbable materials. In addition, bioactive compositions can be incorporated in these materials.

Preferred metallic materials are alloys of nickel and titanium having superelastic (SE) properties. These superelastic metallic alloys are commonly referred to as nitinol metal. Other metallic materials suitable for use in the present invention include stainless steel, titanium, Elgiloy Specialty Metal (ESM), tantaum, and cobalt-chromium.

Suitable non-bioabsorbable polymeric materials include, but are not limited to, polyurethanes, polyolefins, including fluoropolymers, polyesters, poly(meth)acrylates, polyvinyl fluorides, nylons and combinations thereof. Suitable polymers include but are not limited to polymers selected from the group consisting of polyolefins (such as polyethylene and polypropylene including atactic, isotactic, syndlotactic, and blends thereof as well as, polyisobutylene and ethylene-alphaolefin copolymers); polyesters (such as polyethylene terephthalate and polybutylene terephthalate); acrylic polymers and copolymers, vinyl halide polymers and copolymers (such as polyvinyl chloride); polyvinyl ethers (such as polyvinyl methyl ether); polyvinylidene halides (such as polyvinylidene fluoride and polyvinylidene chloride); polyacrylonitrile; polyvinyl ketones; polyvinyl aromatics (such as polystyrene); polyvinyl esters (such as polyvinyl acetate); copolymers of vinyl monomers with each other and olefins, (such as etheylene-methyl methacrylate copolymers, acrylonitrile-styrene copolymers, ABS resins and ethylene-vinyl acetate copolymers); polyamides (such as nylon 4, nylon 6, nylon 66, nylon 610, nylon 11, nylon 12 and polycaprolactam); alkyd resins; polycarbonates; polyoxymethylenes; polyimides; polyethers; epoxy resins, polyurethanes; rayon; and rayon-triacetate, polyethylene, polypropylene, and thermoplastic copolymers of tetrafluoroethylene and perfluoromethyl vinyl ether, and elastomeric versions thereof. Preferred non-bioabsorbable materials are fluoropolymer-based materials.

Suitable bioabsorbable materials suitable for use in the present invention include, but are not limited to, polyglycolic acid - trimethylenecarbonate co-polymers, aliphatic polyesters, poly(amino acids), copoly(ether-esters), polyalkylenes oxalates, polyamides, poly(iminocarbonates), polyorthoesters, polyoxaesters, polyamidoesters, polyoxaesters containing amido groups, poly(anhydrides), polyphosphazenes, and blends thereof. For the purpose of this invention aliphatic polyesters include, but are not limited to, homopolymers and copolymers of lactide (which includes D- and L- lactic acids; D-, L-, and meso lactide), glycolide (including glycolic acid), epsilon-caprolactone, p-dioxanone (1,4-dioxan-2-one), trimethylene carbonate (1,3-dioxan-2-one), alkyl derivatives of trimethylene carbonate, delta-valerolactone, beta-butyrolactone, gamma-butyrolactone, epsilon-decalactone, hydroxybutyrate, hydroxyvalerate, alpha.,.alpha.-diethylpropiolactone, ethylene carbonate, ethylene oxalate, 3-methyl-1,4-dioxan-2,5-dione, 3,3-diethyle-1,4-dioxan-2,5-dione, 6,8-dioxabicycloctane-7-one, 2,5-diketomorpholine, 1,4-dioxepan-2-one (including its dimer 1,5,8,12-tetraoxacyclotetradecane-7,14-dione), 1,5-dioxepan-2-one, 6.6-dimethyl-1,4-dioxan-2-one and polymer blends thereof. Poly(iminocarbonate) for the purpose of this invention include as described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described in "Journal of Biomaterials Research", Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyalkylene oxalates for the purpose of this invention include U.S. Pat. Nos. 4;208,511; 4,141,087; 4,130,639; 4,140,678; 4,105,034; and 4,205,399. Polyphosphazenes copolymers (such as co-, ter- and higher order mixed monomer based polymers) made with L-lactide, D-lactide, meso-lactide, L-lactic acid, D-lactic acid, glycolide, glycolic acid, paradioxanone, trimethylene carbonate and .epsiton-caprolactone such as are described by Allcock in The Encyclopedia of Polymer Science, Vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182 (which are hereby incorporated by reference herein). Polyanhydrides from diacids of the form HOOC--C₆ H₄ --O--(CH₂)ₘ --O--C₆ H₄ --COOH where 'm' is an integer in the range of from 2 to 8 and copolymers thereof with aliphatic alpha-omega diacids of up to 12 carbons. Polyoxaesters polyoxaamides and polyoxaesters containing amines and/or amido groups are described in one or more of the following U.S. Pat. Nos. 5,464,929; 5,595,751; 5,597,579; 5,607,687; 5,618,552; 5,620,698; 5,645,850; 5,648,088; 5,698,213; and 5,700,583. Polyorthoesters such as those described by Heller in Handbook of Biodegradable Polymers, edited by Domb, Kost and Wisemen, Hardwood Academic Press, 1997, pages 99-118. Preferred bioabsorbable polymeric materials include polyglycolic acid - trimethylene carbonate (PGA:TMC) block co-polymeric materials.

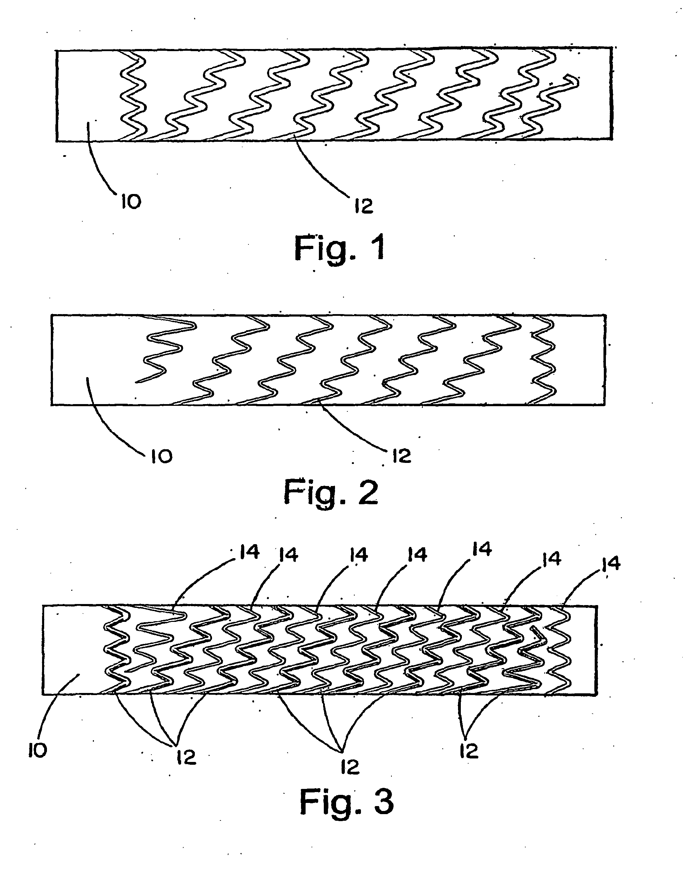

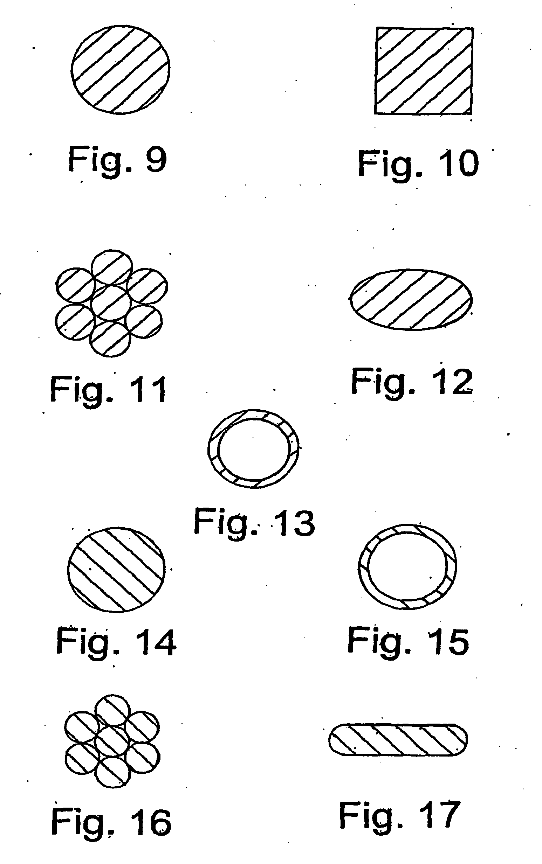

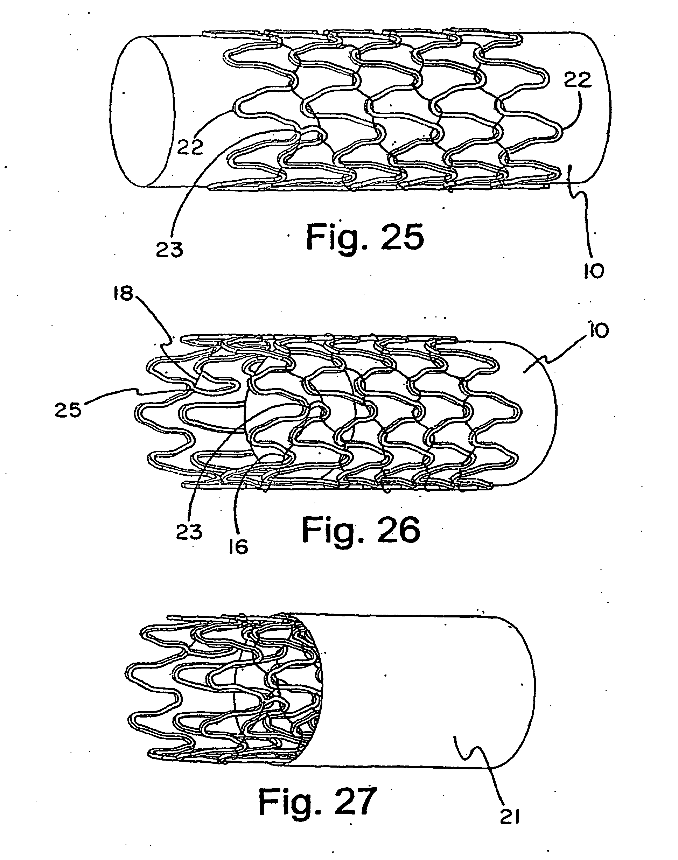

In addition to composition, the dimensions and shape of the flexible framework elements can vary from device to device or from framework element to framework element These dimensions include the length, thickness, or cross-sectional shape of the framework element (*e.g*., Figures 9-13). While the overall shape of the flexible framework elements follows a pattern having an alternating series of oppositely oriented apices, or vertices, along most or all of the length of the framework element (Figures 3-7), the angle at which adjacent portions of the framework elements form the apexes, or vertices, is variable. Furthermore, the sections of framework element located between apices can be curved, straight, or other suitable shape (*e.g.*, Figure 25). Collectively, the angles of the apices, or vertices, are selected so an additional helically disposed flexible framework element can be placed next to a first flexible helically disposed framework element in such a way that apices of the first flexible framework element are adjacent apices of the additional flexible framework element (*e.g.*, Figure 3).

The apices, or vertices, of the flexible framework elements can have a variety of shapes. The shapes can be simple reverse-direction curves, angled turns in the pattern, or combinations thereof. In addition, eyelets and other shapes incorporating an open circular configuration can form an apex or vertex in the flexible framework elements of the present invention.

Two flexible linkage elements (16.18) are used for each pair of flexible framework elements (Figure 6). Each flexible linkage element is threaded through the first and second flexible framework elements on a pathway, or course, separate and distinct from other flexible linkage elements along all or part of the length of the invention (Figures 4 and 5). As with the flexible framework elements, the flexible linkage elements can have a variety of cross-sectional shapes and areas (*e.g.*, Figures 14-17).

Generally, a length of a first flexible linkage element (16) is attached to a first flexible framework element (12). The first flexible linkage element is threaded over (or under) a portion of the first flexible framework element adjacent to, or forming part of, an apex in the first framework element (Figure 4). The first flexible linkage element is then threaded under (or over, respectively) a nearby portion of a second flexible framework element adjacent to, or forming part of, an apex in the second flexible framework element (Figure 4). The first flexible linkage element is threaded in this "over-and-under" process along all or part of the length of the invention to form a first course. A second flexible linkage element-(18) is similarly threaded from a second flexible framework element (14) through the first flexible framework along a second course separate and distinct from the first course (Figure 5). In some embodiments, it may be necessary to thread a first flexible linkage element through several "apex-forming portions" of a first flexible framework element until a portion of a second flexible framework element is encountered appropriate for the beginning of a first course. At a terminus of the first course, the first flexible linkage element may need to be threaded through several "apex-forming portions" of the first flexible framework elements, as well. A similar arrangement for a second course is often employed.

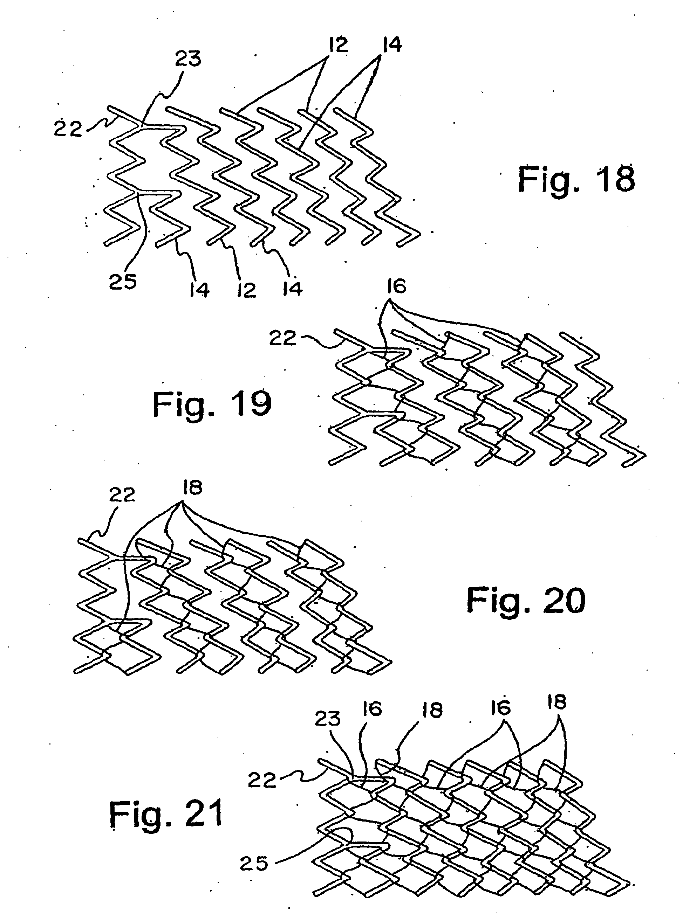

In a preferred embodiment, first flexible framework elements and second flexible framework elements are both attached to a common flexible framework element (22) located at one or both ends of the invention (Figures 18 - 21 and 25 - 27). In these embodiments, the first flexible framework element and the second flexible framework element each have terminal ends. The first and second flexible framework elements each have terminal ends that are individually attached to the common framework element. In some embodiments, the common framework element is incorporated into, or otherwise continuous with, the flexible framework elements (Figure 18). The common framework element eliminates some or all of the free ends of the flexible framework elements. Common framework elements can be made of any suitable non-bioabsorbable material and/or bioabsorbable material, including those materials listed herein. The cross-section, shape, configuration, and/or conformation of a common framework element can be similar to those of the flexible framework elements or they can be of another design. Inclusion of at least one common framework element with at least two flexible framework elements is readily accomplished by cutting an appropriate material according to a pattern.

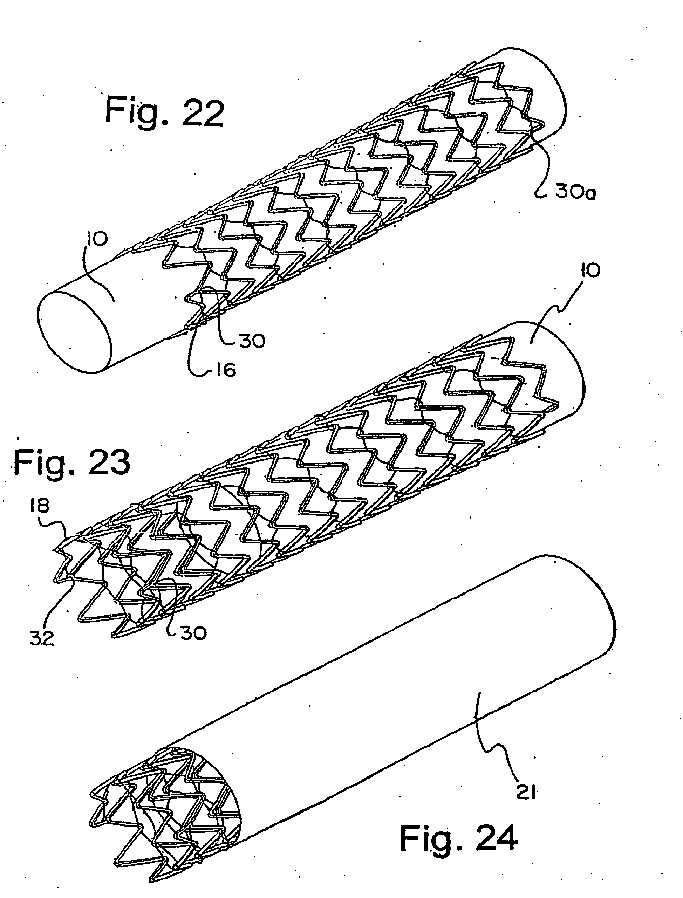

In another preferred embodiment, a first flexible framework element is attached directly to a second flexible framework element, without the use of a common framework element (Figures 22 - 24). In these embodiments, a first flexible linkage element (16) is attached to junction (30) of the first flexible framework element and the second flexible framework element The first flexible linkage element is then threaded through the most proximate apex-forming portion of the first flexible framework element (12). The first flexible linkage element is then threaded over, or under, the most proximate apex-forming portion of the second flexible framework element to begin a first course. The first flexible linkage element is alternately passed through the apices of the first flexible framework element and the second flexible framework element to form a first course. The first course is terminated by attaching the first flexible linkage element to junction (30a) of the first flexible framework element and the second flexible framework element.

Similarly, a second flexible linkage element (18) is attached to junction (32) of the second flexible framework element and the first flexible framework element The second flexible linkage element is then threaded through the most proximate apex-forming portion of the second flexible framework element (14). The second flexible linkage element is then threaded over, or under, the most proximate apex-forming portion of the first flexible framework element to begin a second course. The second flexible linkage element is alternately passed through the apices of the second flexible framework element and the first flexible framework element to form a second course. The second course is terminated by attaching the second flexible linkage element to a junction of the second flexible framework element and the first flexible framework element.

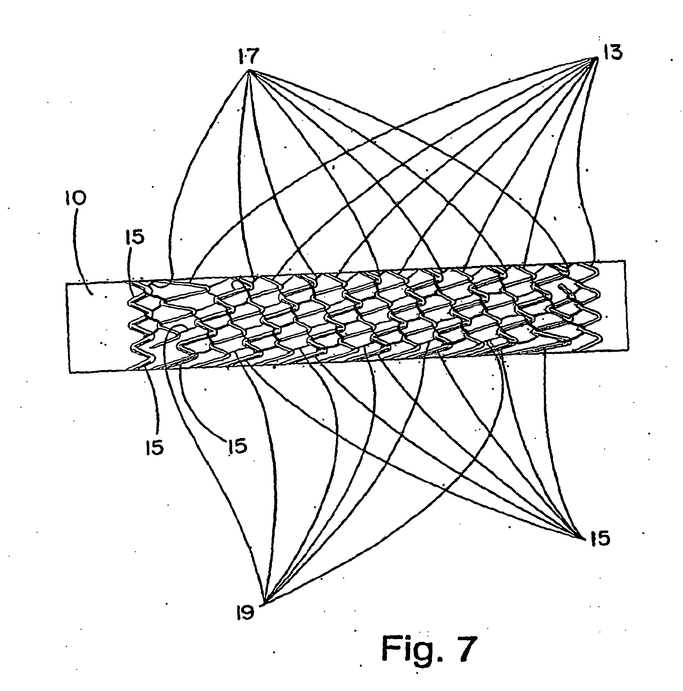

When the invention is in an extended, uncollapsed, configuration, the first flexible linkage element and the second flexible linkage element tend to be located in the respective apices of the first flexible framework element and the second flexible framework element (Figure 7). Connecting adjacent apices of separate flexible framework elements with flexible linkage elements following distinct courses imparts a high degree of mechanical flexibility, stability, and material variability to the invention. If desired, additional flexible framework elements and flexible linkage elements are included in the same fashion.

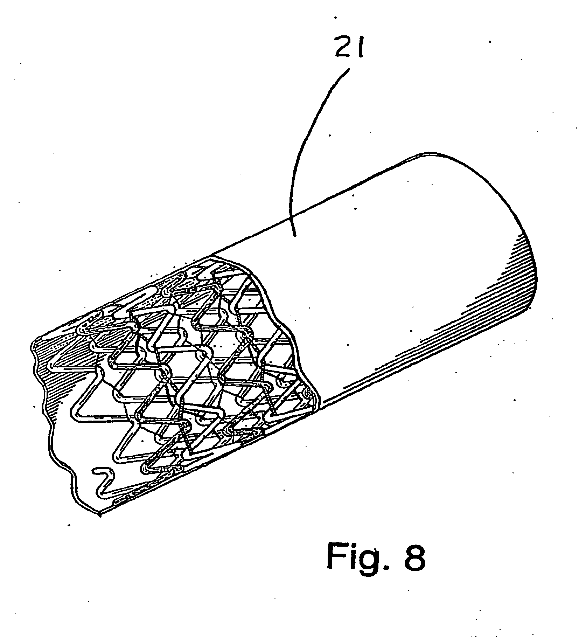

The flexible elements of the present invention can be covered or coated with materials or substances that enhance biocompatibility, mechanical interaction of the elements, and/or resistance to thrombogenesis. The coating material or coating substance can also alter the absorption rate(s) of the bioabsorbable portion(s) of the invention. Materials suitable for covering the flexible elements are listed herein above. These materials can be applied In the form of extruded tubes, film wraps, powder coatings, and spray coatings. Suitable materials for coating flexible elements of the present invention included the compounds listed herein above. A preferred covering material (21) for constructing a stent-graft of the present invention (Figure 8) is porous expanded polytetrafluoroethylene (ePTFE).

Coverings and/or coatings placed on all or part of the flexible elements of the present invention can contain biologically active substances or entities. Preferred biologically active substances reduce or inhibit thrombus formation on the invention. Heparin, heparin analogs and derivatives are particularly preferred anti-thrombotic agents for use in the present invention. Other preferred biologically active substances reduce undesirable cellular growth in and around tissue in which the present invention is deployed and implanted. A preferred anti-proliferative agent for use in the present invention is dexamethasone. Other biologically active substances suitable for use in the present invention include enzymes, organic catalysts, ribozymes, organometallics, proteins, glycoproteins, peptides, polyamino acids, antibodies, nucleic acids, steroidal molecules, antibiotics, anti-inflammatories, antimycotics, cytokines, carbohydrates, oleophobics, lipids, extracellular matrix material and/or its individual components, pharmaceuticals, and therapeutics. Biological entities suitable for use in the present invention include mammalian cells, including genetically engineered cells, viruses, virenos, prions, and organelles, such as mitochondria.

When used as an implantable medical device within a body conduit or luminal space, a delivery system as described in U.S. Patent Nos. 6, 827,731 and 6,899,727 are preferably used to deliver and deploy the present invention.

### EXAMPLES

### Example 1

This example describes the construction of a device of the present invention using a superelastic nitinol metal alloy. A length of superelastic (SE) nitinol wire having a diameter of 0.178mm (0.007inches) was obtained from Nitinol Devices and Components (Freemont, CA) and used to construct both framework elements.

A first framework element was made by helically winding a first nitinol wire around a stainless steel "stent-jig" (10) having a central axis running the length of the jig. The jig also had a series of pins, approximately 0.5mm in diameter, projecting from the surface of the jig. The jig had a diameter of approximately 7mm. The pins were laid out so the finished helically disposed stent (12) had a pitch angle great enough to nest a second helically disposed stent therewithin (Figure 1). The combined jig and helically disposed stent were subjected to a thermal cycle for 10 minutes in a convection oven (Carbolite, Watertown, WI) set at 450°C. Heating was followed by quenching the combination in distilled water at ambient temperature. Alternatively, the construction was subjected to a thermal cycle sufficient to set the austenite finish temperature to approximately 37°C.

A second framework element was made by helically winding a second nitinol wire around a stainless steel "stent-jig" (10) having a central axis running the length of the jig. The jig also had a series of pins (not shown) projecting from the surface thereof. The pins had a diameter of approximately 6.7mm. The pins were laid out so the finished helically disposed stent (14) had a pitch angle sufficient to permit the second helical winding to nest within the first framework element. The combined jig and helically disposed stent were subjected to a thermal cycle for 10 minutes in a convection oven (Carbolite, Watertown, WI) set at 450°C. Heating was followed by quenching the combination in distilled water at ambient temperature.

As seen in Figure 3, the first framework element and the second framework element were both helically disposed around a stainless steel mandrel (10) having a common central axis. The diameter of the mandrel was approximately 7mm (0.275inch). The framework elements were rotated with respect to one another on the mandrel until apices from the first framework element (12) were aligned adjacent to apices from the second framework element (14).

A first flexible linkage element (16) was formed from an ePTFE fiber (CV-5 Suture, W.L. Gore & Associates, Inc., Flagstaff, AZ) by tying the first-flexible linkage element to one terminus of the first framework element and weaving the first flexible linkage element between adjacent apices of the first framework element and the second framework element in a helical fashion to form a first course (Figure 4). At the end of the first course, the first flexible linkage element was tied to the first framework element at the opposite terminus of the first framework element.

A second flexible linkage element (18) was formed from an ePTFE fiber (CV-5 Suture, W.L. Gore & Associates, Inc., Flagstaff, AZ) by tying the second flexible linkage element to one terminus of the second framework element and weaving the second flexible linkage element between adjacent apices of the first framework element and the second framework element in a helical fashion to form a second course distinct for the first course (Figure 5). In Figure 5, the first flexible linkage element has been deleted for clarity. At the end of the second course, the second flexible linkage element was tied to the second framework element at the opposite terminus of the second framework element. The finished device was then removed from the mandrel.

A side view of the completed device is illustrated in Figure 6.

### Example 2

This example describes the construction of an embodiment of the present invention having two framework elements made of a bioabsorbable material. The two flexible linkage elements were also made of a bioabsorbable material.

A first framework element (13) was made of a polyglycolic acid - trimethylene carbonate (PGA:TMC) co-polymeric material. The PGA:TMC material was obtained in the form of an extruded monofilament from U.S. Surgical (Norwalk, CT) having a diameter of 0.38mm (0.015 inches). The monofilament had been stored under refrigeration.

The polymeric mono-filament was wound onto a first stainless steel "stent-jig" made of a stainless steel mandrel (10) and having pins projecting outwardly from the surface of the mandrel. The pins had a diameter of approximately 6.7mm. The pins (not shown) were arranged in a pattern on the mandrel so the resulting first framework element had a pitch angle great enough to permit nesting of a second framework element within the first framework element (Figure 7).

Once wound on the jig, the PGA:TMC mono-filamentous material was subjected to a heating and cooling cycle in a convection oven set at 150°C for ten minutes and allowed to cool to room temperature in air. The heating and cooling cycle set the first framework element in the shape of a helix disposed around a central axis that will be in common with a second framework element. The helical shape of the first framework element was retained after the framework element was removed from the stent-jig.

Another polymeric PGA:TMC monofilament of similar dimensions was wound around a second stainless steel "stent-jig." The second stent-jig was similar to the first stent-jig except for the placement of the pins on the mandrel portion of the jig. In the second stent-jig, the pins were arranged in a pattern that complemented the pattern and pitch angle of the pins on the first stent-jig so the resulting second framework element (15) would wind around the same central axis as the first framework element so that apices of the first framework element and apices of the second framework element are located adjacent to one another. This second PGA:TMC mono-filamentous material was subjected to the same heating and cooling cycle as the first framework element. The resulting helically disposed second framework element was removed from the stent-jig and placed on a stainless steel mandrel (diameter 7mm) along with the first framework element. The two framework elements were rotated with respect to one another on the mandrel until the apices of the first framework element and apices of the second framework element were located adjacent to one another. The "nested" framework elements were connected with two separate flexible linkage elements (Figure 7).

The first flexible linkage element (17) was in the form of a fiber made of a polymeric bioabsorbable PGA:TMC material. The fiber obtained for this example was a Maxon Suture (Davis & Geck, Inc.). The first flexible linkage element was initially tied to one terminus of the first framework element and threaded over a portion of a linkage element forming an apex and under a portion of the linkage element forming the same apex. The first flexible linkage element was threaded through adjacent apices of the first framework element in the same manner until an apex of the second framework element was encountered. At that point, the first flexible linkage element was threaded from the first framework element over (or under) a portion of the second framework element forming the apex, through an space defined by the apex, and under (or over) the opposite portion of the second framework element forming the apex. The first flexible linkage element was threaded through adjacent apices of the "nested" first framework element and the second framework element in a helical fashion to form a first course (Figure 7). The first course of the first flexible linkage continued past the opposite terminus of the second framework element through a series of adjacent apices of the first framework element. At the end of the first course, the first flexible linkage element was tied to the first framework element at the opposite terminus of the first framework element.

A second flexible linkage element (19) was formed from a mono-filamentous bioabsorbable PGA:TMC Maxon Suture by tying the second flexible linkage element to one terminus of the second framework element and weaving the second flexible linkage over and under portions of the first framework element and the second framework element forming adjacent apices. The second flexible linkage element was threaded through the first framework element and the second framework element in a helical fashion to form a second course distinct for the first course (Figure 7). At the end of the second course, the second flexible linkage element was tied to the second framework element at the opposite terminus of the second framework element. The finished device was then removed from the mandrel.

A side view of the completed device is illustrated in Figure 7.

### Example 3

This example describes construction of an embodiment of the present invention having a first framework element made of a metallic material and a second framework element made of a polymeric bioabsorbable material. The first flexible linkage element and the second flexible linkage element were made of a polymeric bioabsorbable material.

In this example, a first framework element made of a superelastic (SE) nitinol wire was constructed as described in Example 1, *supra*. A second framework element made of a polymeric bioabsorbable material was constructed as described in Example 2, *supra.* The two finished framework elements were placed on a 7mm diameter mandrel and rotated with respect to one another on the mandrel until the apices of the first framework element and apices of the second framework element were located adjacent to one another.

The "nested" framework elements were connected with a first flexible linkage element and a second flexible linkage element as described in Examples 1 and 2, *supra.* The flexible linkage elements were each made of a polymeric bioabsorbable PGA:TMC material in the form a Maxon Suture.

### Example 4

This example describes covering at least one metallic framework element with a fluoropolymeric material prior to construction of a device of the present invention. In this example, a length of superelastic (SE) nitinol wire having a diameter of 0.178mm (0.007 inches) was obtained from Nitinol Devices and Components (Freemont, CA) and formed into a framework element as described in Example 1, *supra.* The formed wire was then attached to a machine having rotating chucks that permitted the formed wire to be carefully manipulated into a more straightened wire. This process was conducted in a refrigerated chamber to further manipulate the shape of the formed wire. The framework element retained its "shape-memory" throughout this process.

Once the framework element had been extended, a thin film of expanded porous polytetrafluoroethylene (W.L Gore & Associates, Inc., Flagstaff, AZ) was helically wrapped around the framework elements. After one pass of helical wrap around the extended metallic framework element, the chamber was warmed and the film-wrapped wire was allowed to reorder itself into the serpentine configuration. During this process, the film-wrapped flexible framework element was combined with another flexible framework element to form a tubular stent by rotating both framework elements in the same direction.

### Example 5

This example describes covering at least one metallic framework element with a fluoropolymeric material prior to construction of a device of the present invention. A framework element was prepared and extended as described in Examples 1 and 4, respectively. In this example, an extruded tube of expanded porous polytetrafluoroethylene material having an inner diameter (I.D.) of 0.178 mm (0.007 inches) and an outer diameter (O.D.) of 0.254 mm (0.010 inches) was slid over the framework element. The covered framework element was then combined with another flexible framework element to construct a device of the present invention as described herein elsewhere.

### Example 6

This example describes coating at least one framework element or flexible linkage element with a bioabsorbable material. In this example, a co-polymeric polyglycolic acid - trimethylene carbonate (PGA:TMC) material was dissolved in acetone and sprayed onto the flexible framework or flexible linkage element. Once the acetone solvent had evaporated, the element was used to construct a device of the present invention.

### Example 7

This example describes coating at least one framework element with a bioabsorbable material having at least one biologically active substance releasably incorporated therein. In this example, dexamethasone was added to the PGA:TMC solution described in Example 6 and sprayed onto a flexible element of the present invention. A device of the present invention was constructed using this coated element.

### Example 8

This example describes construction of a covered-stent, or stent-graft, using any of the devices of Examples 1, 4, or 5, *supra,* as generally taught by Martin et al. in U.S. Patent No. 6,520,986.

Referring to Figure 8, a generally tubular stent construction having two helically would flexible framework elements was constructed as described herein, *supra*. The generally tubular flexible framework was inserted inside a generally tubular graft member (21) having an inner diameter sufficient to contact outer surfaces of at least one of the flexible framework elements. The tubular graft member was made of an expanded polytetrafluoroethylene material. A coupling member was used to attach the at least one flexible framework element to the graft member. The coupling member was in the form of a ribbon and covered only a portion of each flexible framework element. With this construction, regions of the flexible framework elements did not contact the coupling member.

### Example 9

This example describes construction of an embodiment of the present invention in a tubular configuration having a common framework element incorporated at each end of the construction. The framework elements were metallic and the linkage elements were fluoropolymeric In composition. The metallic elements were formed by laser cutting a tubular piece of stainless steel according to a pattern. An uncut cylindrical piece of stainless steel was placed on a mandrel (10) for laser cutting. Figures 41 and 44 are representative examples of flexible framework elements of the present invention that can be formed with patterned laser cutting.

Figures 18 - 21 are schematic representations showing one end of a tubular construction that had been cut open longitudinally and flattened. Although only one common end is shown in these figures, both ends of the tubular construction of this example had a common framework element.

As shown in Figure 18, one end of the first flexible framework element (12) was attached to common framework element (22) at site (23) and one end of the second flexible framework element (14) was attached to common framework element at site (25). Similarly, one end of the second flexible framework element (14) was attached to common framework element (22) at site (25). In practice, a common framework element was also placed at the opposite end of the construction in the same fashion.

As shown in Figure 19, a first flexible linkage element (16) was attached to the construction at site (23) and threaded through an apex of the first flexible framework element (12) most proximate to the attachment site (23). In this embodiment, the first flexible linkage element (16) was threaded through an apex of the common framework element adjacent the attachment site (23). The first flexible linkage element (16) was then threaded through the next available apex of the first framework element (12). Next, the first flexible linkage element (16) was threaded through the apex of the second flexible framework element (14) most proximate to the previous apex of the first flexible framework element to form the beginning of a first course. The first flexible linkage element (16) was alternately threaded through apices of the first flexible framework element and the second flexible framework element as described elsewhere herein to complete the first course. The first course was terminated by attaching the first flexible linkage to a common framework element at the opposite end of the construction in a similar manner.

As shown in Figure 20, a second flexible linkage element (18) was attached to the construction at site (25) and threaded through an apex of the second flexible framework element (14) most proximate to the attachment site (25). In this embodiment, the second flexible linkage element (18) was threaded through an apex of the common framework element adjacent the attachment site (25). The second flexible linkage element (16) was then threaded through the next available apex of the second framework element (14). Next, the second flexible linkage element (18) was threaded through the apex of the first flexible framework element (12) most proximate to the previous apex of the second flexible framework element to form the beginning of a second course. The second flexible linkage element (18) was alternately threaded through apices of the second flexible framework element and the first flexible framework element as described elsewhere herein to complete the second course. The second course was terminated by attaching the second flexible linkage to a common framework element at the opposite end of the construction in a similar manner.

Figure 21 illustrates each of the elements of this embodiment in relation to one another.

Figures 25 - 27 illustrate tubular embodiments of the present invention having a common framework element (22) at both ends of the tubular construction. The framework elements (12, 14) in these embodiments are characterized by the absence of any sections that are straight. Every section of every framework element (12, 14, 22) was curved.

Figure 25 illustrates a stent of the present invention placed on a mandrel (10). Figure 26 illustrates the stent of Figure 25 partially withdrawn from a mandrel and flexible linkage elements (16, 18) added. Figure 27 illustrates a stent-graft of the present invention having covering (21) placed over the flexible framework elements and flexible linkage elements illustrated in Figure 26.

### Example 10

This example describes construction of a stent-graft of the present invention. In this example, a tubular stent as described in Example 9, *supra,* was obtained. A porous expanded polytetrafluoroethylene (ePTFE) material in a tubular form of was obtained from W.L. Gore & Associates, Inc., Flagstaff, AZ and slid over the outside of the stent as a covering (21). The covering was attached to the framework elements (12, 14) of the stent with a series of ribbon-like fluoropolymeric materials (not shown). The ribbon-like materials were wrapped around the stent-graft such that apices of the stent were trapped between the ribbon-like material and the covering. The ribbon-like material was attached to the covering though the localized application of heat. In a similar embodiment, the tubular ePTFE material was placed inside the stent as a liner and the framework apices trapped between the liner and an externally applied fluoropolymeric ribbon-like material. The liner and ribbon-like material were attached through the localized application of heat.

### Example 11

This example describes construction of an embodiment of the present invention in which a first flexible framework element (12) was attached to a second flexible framework element (14) without the use of a common framework element (Figures 22 - 24). Figure 22 has second flexible linkage element (18) omitted for clarity. Figure 23 has first flexible linkage element (16) omitted for clarity.

The framework elements were metallic and the linkage elements were fluoropolymeric in composition. The metallic elements were formed by laser cutting a tubular piece of stainless steel according to a pattern. An uncut cylindrical piece of stainless steel was placed on a mandrel (10) for laser cutting.

As shown in Figure 22, first flexible framework element (12) was attached to second framework element (14) at site (30). In Figure 23, second flexible framework element (14) is shown attached to first flexible framework element (12) at site (32).

The first flexible linkage element (16) was attached to the invention at site (30) and threaded through the apex of the first flexible framework element immediately adjacent to attachment site (30). The first flexible framework element was then threaded through the apex of the second flexible framework element immediately adjacent the aforementioned threaded apex of the first flexible framework element to form the beginnings of a first course. The first flexible linkage element was threaded through adjacent apices of the first flexible framework elements and the second flexible framework elements to form the first course. The first course was terminated by attaching the first flexible linkage element to site (30a) at the opposite end of the tubular construction.

The second flexible linkage element (18) was attached to the invention at site (32) and threaded through the apex of the second flexible framework element immediately adjacent to attachment site (32). The second flexible framework element was then threaded through the apex of the first flexible framework element immediately adjacent the aforementioned threaded apex of the second flexible framework element to form the beginnings of a second course. The second flexible linkage element was threaded through adjacent apices of the second flexible framework elements and the first flexible framework elements to form the second course. The second course was terminated by attaching the second flexible linkage element to an attachment site at the opposite end of the tubular construction.

### Example 12

This example describes construction of a stent-graft of the present invention (Figure 24). In this example a tubular stent as described in Example 11, *supra,* was obtained. A porous expanded polytetrafluoroethylene (ePTFE) material in a tubular form of was obtained from W.L. Gore & Associates, Inc., Flagstaff, AZ and slid over the outside of the stent as a covering (21). The covering was attached to the framework elements (12, 14) of the stent with a series of ribbon-like fluoropolymeric materials (not shown). The ribbon-like materials were wrapped around the stent-graft such that apices of the stent were trapped between the ribbon-like material and the covering. The ribbon-like material was attached to the covering though the localized application of heat. In a similar embodiment, the tubular ePTFE material was placed inside the stent as a liner and the framework apices trapped between the liner and an externally applied fluoropolymeric ribbon-like material. The liner and ribbon-like material were attached through the localized application of heat.

## Claims

1. An implantable medical device comprising:
a first framework element (12, 13) having a length and a series of apices oriented in opposite directions along the length of the first framework element (12, 13), wherein said first framework element (12, 13) is helically disposed around a common central axis;
**characterised in that** the implantable medical device further comprises
a second framework element (14, 15) having a length and a series of apices oriented in opposite directions along the length of the second framework element (14, 15), wherein said second framework element (14, 15)is helically disposed around the common central axis and positioned together with the first framework element (12, 13) so that apices of the first framework element (12, 13) and apices of the second framework element (14, 15) are located adjacent to one another;
a first flexible linkage element (16, 17) linking the first framework element (12, 13) to the second framework element (14, 15) along a first course; and
a second flexible linkage element (18, 19) linking the first framework element (12, 13) to the second framework element (14, 15) along a second course distinct from the first course.

2. The implantable medical device of claim 1 further comprising a covering (21) placed on at least a portion of said device.

3. The implantable medical device of claim 2 further comprising a bioactive material releasably combined with said covering (21).

4. The implantable medical device of claim 1 or 2 wherein the first flexible linkage element (16, 17) connects at least two adjacent apices of the first framework element (12, 13) and the second framework element (14, 15).

5. The implantable medical device of claim 1 or 2 wherein the second flexible linkage element (18, 19) connects at least two adjacent apices of the first framework element (12, 13) and the second framework element (14, 15).

6. The implantable medical device of claim 1 or 2 wherein the first framework element (12, 13) is made of a material exhibiting different physical properties than those of the second framework element (14, 15).

7. The implantable medical device of claim 1 or 2 wherein the first flexible linkage element (16, 17) is made of a material exhibiting different physical properties than those of the second flexible linkage element (18, 19).

8. The implantable medical device of claim 1 or 2 wherein the first framework element (12, 13) has a cross-sectional profile exhibiting different physical properties than those of the second framework element (14, 15).

9. The implantable medical device of claim 1 or 2 wherein the first flexible linkage element (16, 17) has a cross-sectional profile exhibiting different physical properties than those of the second flexible linkage element (18, 19).

10. The implantable medical device of claim 1 or 2 further comprising a third framework element (22) having a length and a series of apices oriented in opposite directions along the length of the third framework element (22).

11. The implantable medical device of claim 10 further comprising a third flexible linkage element.

## Patentansprüche

1. Implantierbare medizinische Vorrichtung umfassend:
ein erstes Strukturelement (12, 13), das eine Länge und eine Reihe von Spitzen aufweist, die in entgegengesetzten Richtungen der Länge des ersten Strukturelements (12, 13) entlang orientiert sind, wobei das erste Strukturelement (12, 13) spiralenförmig um eine gemeinsame mittlere Achse angeordnet ist;
**dadurch gekennzeichnet, dass** die implantierbare medizinische Vorrichtung des Weiteren Folgendes umfasst
ein zweites Strukturelement (14, 15), das eine Länge und eine Reihe von Spitzen aufweist, die in entgegengesetzten Richtungen der Länge des zweiten Strukturelements (14, 15) entlang orientiert sind, wobei das zweite Strukturelement (14, 15) spiralenförmig um die gemeinsame mittlere Achse angeordnet und zusammen mit dem ersten Strukturelement (12, 13) so positioniert ist, dass Spitzen des ersten Strukturelements (12, 13) und Spitzen des zweiten Strukturelements (14, 15) sich nebeneinander befinden;
ein erstes flexibles Verbindungselement (16, 17), das das erste Strukturelement (12, 13) mit dem zweiten Strukturelement (14, 15) einer ersten Bahn entlang verbindet; und
ein zweites flexibles Verbindungselement (18, 19), das das erste Strukturelement (12, 13) mit dem zweiten Strukturelement (14, 15) einer zweiten Bahn entlang, die von der ersten Bahn verschieden ist, verbindet.

2. Implantierbare medizinische Vorrichtung nach Anspruch 1, des Weiteren eine Bedeckung (21) umfassend, die auf mindestens einen Abschnitt der Vorrichtung aufgebracht ist.

3. Implantierbare medizinische Vorrichtung nach Anspruch 2, des Weiteren ein bioaktives Material umfassend, das freisetzbar mit der Bedeckung (21) kombiniert ist.

4. Implantierbare medizinische Vorrichtung nach Anspruch 1 oder 2, wobei das erste flexible Verbindungselement (16, 17) mindestens zwei nebeneinanderliegende Spitzen des ersten Strukturelements (12, 13) und des zweiten Strukturelements (14, 15) verbindet.

5. Implantierbare medizinische Vorrichtung nach Anspruch 1 oder 2, wobei das zweite flexible Verbindungselement (18, 19) mindestens zwei nebeneinanderliegende Spitzen des ersten Strukturelements (12, 13) und des zweiten Strukturelements (14, 15) verbindet.

6. Implantierbare medizinische Vorrichtung nach Anspruch 1 oder 2, wobei das erste Strukturelement (12, 13) aus einem Material besteht, das andere physikalische Eigenschaften als diejenigen des zweiten Strukturelements (14, 15) aufweist.

7. Implantierbare medizinische Vorrichtung nach Anspruch 1 oder 2, wobei das erste flexible Verbindungselement (16, 17) aus einem Material besteht, das andere physikalische Eigenschaften als diejenigen des zweiten flexiblen Verbindungselements (18, 19) aufweist.

8. Implantierbare medizinische Vorrichtung nach Anspruch 1 oder 2, wobei das erste flexible Strukturelement (12, 13) ein Querschnittsprofil aufweist, das andere physikalische Eigenschaften als diejenigen des zweiten Strukturelements (14, 15) aufweist.

9. Implantierbare medizinische Vorrichtung nach Anspruch 1 oder 2, wobei das erste flexible Verbindungselement (16, 17) ein Querschnittsprofil aufweist, das andere physikalische Eigenschaften als diejenigen des zweiten flexiblen Verbindungselements (18, 19) aufweist.

10. Implantierbare medizinische Vorrichtung nach Anspruch 1 oder 2, des Weiteren ein drittes Strukturelement (22) umfassend, das eine Länge und eine Reihe von Spitzen aufweist, die in entgegengesetzten Richtungen der Länge des dritten Strukturelements (22) entlang orientiert sind.

11. Implantierbare medizinische Vorrichtung nach Anspruch 10, des Weiteren ein drittes flexibles Verbindungselement umfassend.

## Revendications

1. Dispositif médical implantable comprenant :
un premier élément formant cadre (12, 13) ayant une longueur et une série d'apex orientés dans des sens opposés le long de la longueur du premier élément formant cadre (12, 13), ledit premier élément formant cadre (12, 13) étant disposé de manière hélicoïdale autour d'un axe central commun ;
**caractérisé en ce que** le dispositif médical implantable comprend en outre
un second élément formant cadre (14, 15) ayant une longueur et une série d'apex orientés dans des sens opposés le long de la longueur du second élément formant cadre (14, 15), ledit second élément formant cadre (14, 15) étant disposé de manière hélicoïdale autour de l'axe central commun et positionné conjointement au premier élément formant cadre (12, 13) de sorte que les apex du premier élément formant cadre (12, 13) et les apex du second élément formant cadre (14, 15) soient situés de manière adjacente les uns par rapport aux autres ;
un premier élément formant lien souple (16, 17) liant le premier élément formant cadre (12, 13) au second élément formant cadre (14, 15) le long d'un premier déplacement ; et
un second élément formant lien souple (18, 19) liant le premier élément formant cadre (12, 13) au second élément formant cadre (14, 15) le long d'un second déplacement distinct du premier déplacement.

2. Dispositif médical implantable selon la revendication 1, comprenant en outre un recouvrement (21) placé sur au moins une partie dudit dispositif.

3. Dispositif médical implantable selon la revendication 2, comprenant en outre un matériau biologiquement actif combiné de manière amovible audit recouvrement (21).

4. Dispositif médical implantable selon la revendication 1 ou 2, le premier élément formant lien souple (16, 17) étant connecté à au moins deux apex adjacents du premier élément formant cadre (12, 13) et du second élément formant cadre (14, 15).

5. Dispositif médical implantable selon la revendication 1 ou 2, le second élément formant lien souple (18, 19) relie au moins deux apex adjacents du premier élément formant cadre (12, 13) et du second élément formant cadre (14, 15).

6. Dispositif médical implantable selon la revendication 1 ou 2, le premier élément formant cadre (12, 13) étant fabriqué à partir d'un matériau faisant preuve de différentes propriétés physiques par rapport à celles du second élément formant cadre (14, 15).

7. Dispositif médical implantable selon la revendication 1 ou 2, le premier élément formant lien souple (16, 17) étant fabriqué à partir d'un matériau faisant preuve de différentes propriétés physiques par rapport à celles du second élément formant lien souple (18, 19).

8. Dispositif médical implantable selon la revendication 1 ou 2, le premier élément formant cadre (12, 13) ayant un profil transversal faisant preuve de différentes propriétés physiques par rapport à celles du second élément formant cadre (14, 15).

9. Dispositif médical implantable selon la revendication 1 ou 2, le premier élément formant lien souple (16, 17) ayant un profil transversal faisant preuve de différentes propriétés physiques par rapport à celles du second élément formant lien souple (18, 19).

10. Dispositif médical implantable selon la revendication 1 ou 2, comprenant en outre un troisième élément formant cadre (22) ayant une longueur et une série d'apex orientés dans des sens opposés par rapport à la longueur du troisième élément formant cadre (22).

11. Dispositif médical implantable selon la revendication 10, comprenant en outre un troisième élément formant lien souple.
